# EUROPEAN PATENT SPECIFICATION

(11) **EP 3 485 876 B1**
(45) Date of publication and mention of the grant of the patent: **22.11.2023**
(21) Application number: 17802796.7
(22) Date of filing: 23.05.2017
(51) Int. Cl.: A61K 9/50, A61K 9/14, A61K 9/16, A61K 9/48, A61K 47/08, A61K 47/10, A61K 47/12, A61K 47/14, A61K 47/26, A61K 47/36, A61K 47/44, A61K 47/42, A61K 31/436, A61K 38/13, A61K 31/55, A61K 31/706, A61P 25/08, A61P 37/06, A61P 35/00

(54) **ORAL PHARMACEUTICAL COMPOSITION AND METHOD FOR PRODUCING PARTICULATE FORMULATION COMPRISING COMPOSITION**
ORALE PHARMAZEUTISCHE ZUSAMMENSETZUNG UND VERFAHREN ZUR HERSTELLUNG EINER TEILCHENFÖRMIGEN FORMULIERUNG MIT DER ZUSAMMENSETZUNG
COMPOSITION PHARMACEUTIQUE ORALE ET PROCÉDÉ DE PRODUCTION D'UNE FORMULATION PARTICULAIRE COMPRENANT LA COMPOSITION

(30) Priority: 24.05.2016 JP 2016103212
(43) Date of publication of application: 22.05.2019
(60) Divisional application: 23195426.4
(73) Proprietor: Sunsho Pharmaceutical Co., Ltd., Shizuoka 419-0201 (JP)
(72) Inventor: ENDO, Norimasa, Fujinomiya-shi Shizuoka 418-0013 (JP); HIRASAWA, Wataru, Fujinomiya-shi Shizuoka 418-0013 (JP); MORI, Jun, Fujinomiya-shi Shizuoka 418-0013 (JP); FUKASAWA, Takayuki, Fujinomiya-shi Shizuoka 418-0013 (JP)
(74) Representative: van Breda, Jacobus
(86) International application number: PCT/JP2017/019167
(87) International publication number: WO 2017/204210

(56) References cited:
- WO-A1-2008/053920
- WO-A1-2013/035423
- WO-A2-2010/133609
- JP-A- S5 753 232
- JP-A- 2010 519 200
- JP-A- 2011 241 150
- JP-A- 2012 001 487
- JP-A- 2013 501 756
- US-A1- 2011 052 707
- US-A1- 2014 234 418
- US-B2- 8 088 410

## Description

### TECHNICAL FIELD

The present invention relates to an oral pharmaceutical composition in which dose adjustment is easy, ingestibility is high and dissolution properties are optimized, and that is suitable especially for formulating of drugs having a narrow range of therapeutic drug concentration in the blood, and to a method for producing a particulate formulation thereof.

### BACKGROUND ART

In drug therapy, an amount of drug absorbed in the body sometimes varies greatly due to individual differences of patients, differences in meal content, or the like. Moreover, there are also many drugs with narrow therapeutic range. It is known that it is difficult to stably obtain a sufficient therapeutic effect with these drugs, due to variations in the amount of drug absorbed in a case where drug administration is not precisely and appropriately managed. In these cases, Therapeutic Drug Monitoring (hereinafter abbreviated as TDM) is known as a method for establishing a dosage and administration of the drug depending on the patient's symptoms.

TDM is monitoring the factors related to the therapeutic effects and side effects, while establishing the dosage and administration individualized to each patient. TDM is not necessarily implemented for all drugs, for example,
- drugs with a narrow range of therapeutic drug concentration in the blood and close to a range of drug concentration in which the side effect may occur,
- drugs with large individual differences in in -vivo kinetics,
- drugs with a strong correlation between drug concentration in the blood and expression of drug efficacy/side effects,
- drugs which may cause severe side effects depending on drug
   concentration in the blood, and
- drugs for which it is necessary to carefully check medication adherence,
   are subject to TDM.

Examples of representative drugs for which TDM should be performed include digitalis formulations, theophylline formulations, antiarrhythmic agents, antiepileptic agents, antibiotics (aminoglucoside-based, glycopeptide-based, triazole-based, and the like), immunosuppressants, salicylic acid-based formulations, methotrexate, haloperidol formulations, bromperidol formulations, lithium formulations, imatinib and everolimus (Non Patent Document 1 below). Moreover, dose adjustment individualized to each patient is also required for drugs which have a narrow therapeutic range, a high pharmacological activity and are highly likely to have severe side effects, such as anti-cancer drugs.

In order to establish the dosage and administration individualized to each patient for these drugs, at present commercial formulations are ground (for tablets) or decapsulated (for hard capsules) when dispensing, and shaped and admixed with lactose and the like as needed, then a dose is weighed, and divided and packed by an automatic packing machine. These tasks are cumbersome and inferior in productivity, and may not only decrease efficiency, appropriateness, promptness, timeliness, and the like of the drug therapy, but also expose the dispensing operator to highly pharmacologically active components, which raises concerns in terms of occupational safety. Moreover, when human errors occur during dispensing, changes in the drug's dissolution properties or stability may cause the expected effectiveness to no longer be exhibited and may also lead to a loss in medical benefit for the patient (Non Patent Document 2 below).

Furthermore, many of the patients taking these drugs have a decreased ability to swallow, or have eating/deglutition disorders. For example, epilepsies are reported to be highly prevalent in children and the elderly who have a low ability to swallow, and to have eating/deglutition disorders due to a decrease in mental activity. Patients taking immunosuppressants may have difficulties to eat and swallow, since many are elderly persons of 65 years old or more who have a decreased ability to swallow, and also suffer from severe intraoral defects due to the radiation therapy before and after the organ transplantation. Similarly, anti-cancer drug therapy may cause dry mouth, taste disorder, loss of appetite, nausea and vomiting, and compromised immunity due mainly to the side effects of the drug, and may be accompanied by eating/deglutition disorders. Thus, improvements in the applicability to the medication adherence are more required for drugs for which it is necessary to establish a dosage and administration individualized to each patient, than for conventional drugs (Non Patent Document 3).

On the other hand, many poorly water-soluble drugs are also included among these drugs, so that an improvement of bioavailability associated with improvement of dissolution properties is always considered as an issue, and attempts to solve this issue are being made by application of formulation techniques such as inclusion by solid dispersion, SMEDDS/SEDDS, salt formation, Co-crystal, mechano-fusion and cyclodextrin. However, not only these formulation techniques each have their challenges and problems, but a great amount of excipient is needed to improve the dissolution properties, often causing an enlargement of formulation size, which often required patients to frequently take large-sized formulations. As a result, continuing to take such medication becomes difficult, leading to a loss in medical benefit for patients.

Thus, despite achievement of both medication adherence and bioavailability has always been desired for drugs for which establishing a dosage and administration individualized to each patient is necessary, any successful example, in which the both requirements are achieved, has not yet been found.

As known techniques utilized for improving the medication adherence and the bioavailability, formulating by seamless capsule shown in the following Patent Documents 1 to 8 is known. Conventional seamless capsules are formulations produced by using a double nozzle and adding dropwise a water-soluble film liquid from outer nozzle and an oily content liquid from inner nozzle at the same time to a cooling medium having no compatibility with the aqueous film liquid, and utilizing interfacial tension. Patent Document 1 (JP 5788256 B) discloses a method wherein a water-soluble drug is dissolved in an oily base to form content liquid and made it into a seamless capsule. In the case of poorly water-soluble drugs, when they are dissolved in an oily base in the same way as in Patent Document 1, especially for drugs where the partition coefficient logP is a large positive value, they become trapped in the oily base and can no longer be dissolved, which may cause the amount absorbed in the body to decrease and a loss in therapeutic effect.

Patent Document 2 (JP 5750278 B) discloses a method wherein a multilayer seamless capsule consisting of three layers is produced after dissolving poorly water-soluble drug in a hydrophilic base. However, this method requires special equipment called a triple nozzle and also has problems in terms of productivity such as complication of in-process control, quality control, and the like. Moreover, Patent Document 3 (JP 2002-255793 A) discloses a method to mask unpleasant flavors and odors by dissolving or suspending and mixing a water-soluble component with a strong flavor in a gelatin aqueous solution and forming particles. However, this is not a method intended to adjust dissolution properties of drug.

Moreover, Patent Document 4 (JP 5640079 B) discloses a method adjusting the dissolution properties by shaping emulsified poorly water-soluble drug into solid mini beads (i.e., particulate formulation). However, use of hydrophilic anionic surfactants to improve dissolution properties is essential. On the other hand, Patent Document 5 (International Publication No. 2013/035423) discloses a method shaping poorly water-soluble drug candesartan cilexetil into a solid particulate formulation without using surfactants, and it describes that a good dissolution properties were obtained. However, when the present inventors did a replication study, they confirmed that these dissolution properties were due to the contribution of polysorbate 20 (i.e., hydrophilic nonionic surfactant) contained in the dissolution test liquid, and that the contribution of the formulation's composition was small.

Meanwhile, the reality is that multiple drugs are taken as therapeutic agents for lifestyle-related diseases including antihypertensive agents, and healthcare professionals and patients want a decrease in the number of drugs taken by a combination product. From this viewpoint, Patent Documents 2 and 5 disclose that dosage adjustment becomes easier and that the ingestibility is also improved by encapsulating candesartan cilexetil, which is an antihypertensive agent, in seamless capsules and dividing it into many granular capsules. However, dividing it into many granular capsules goes against the above-described needs.

Patent Document 6 (JPWO 2008/081829 A) discloses a method improving bioavailability by utilizing solid dispersion techniques. Solid dispersion technique is a method making a drug amorphous and dispersing it in a carrier, and examples of main production methods include spray-drying method and heating fusion method. However, these production methods all make production process cumbersome and increase man-hours, causing an increase in cost, and also raising concerns about content reduction, increase of analogous substances and decrease in dissolution properties over time, and the like. When using an organic solvent with spray-drying method, there are concerns over residual solvent, and when using heating fusion method, there are issues such as needing a special kneading device.

In the above Patent Document 4 or Patent Document 7 (JP 5567909 B) and Patent Document 8 (JP 5491724 B), a method improving bioavailability by utilizing a self-emulsifying technique, is disclosed and relatively highly irritating surfactants such as polyoxyl 35 castor oil or polysorbate 80 are used. Moreover, Patent Document 7 discloses that extremely good dissolution properties were obtained compared to a standard formulation (i.e., Astellas Pharma Inc.'s "Prograf Capsules 5 mg"). However, since this does not conform to dissolution properties of standard formulations defined in the "Guideline for Bioequivalence Studies of Generic Products," it does not satisfy the regulations as a generic product, and applicability as a formulation used in actual treatments is unclear.
In patent Document 9 (WO 2010/133609 A2) a composition is disclosed comprising a water-soluble polymer matrix material in which droplets of oil are dispersed. Document 9 specifically teaches that a lower amount of surfactant can be used if the surfactant is incorporated into the composition. In fact, incorporation of (much lower overall quantities of) surfactant in one embodiment of mini-beads of the invention according to Document 9 produces unexpectedly superior in-vivo performance.

### PRIOR ART DOCUMENTS

### PATENT DOCUMENT

Patent Document 1: JP 5788256 B
Patent Document 2: JP 5750278 B
Patent Document 3: JP 2002-255793 A
Patent Document 4: JP 5640079 B
Patent Document 5: WO 2013/035423
Patent Document 6: JPWO 2008/081829 A
Patent Document 7: JP 5567909 B
Patent Document 8: JP 5491724 B
Patent Document 9: WO 2010/133609 A2

### NON PATENT DOCUMENT

Non Patent Document 1: "As of the issues to be concerned regarding implementation accompanying partial amendment of the calculation method of medical fees (Notice) Attachment 1 (Medical points table)" March 5th, 2012, Hoihatsu 0305 No. 1
Non Patent Document 2: Effective Preparation Method for Uniform Packages of 0.1 mg of Solid Dispersion Formulation of Tacrolimus, Jumpei Saito et al., The archives of practical pharmacy Vol. 76 (2016)
Non Patent Document 3: "Drug administration for dysphagia patients" Tokiko Ikegawa of Tomita Pharmaceutical, Ajisai Vol. 16 (2007)

### SUMMARY OF INVENTION

### TECHNICAL PROBLEM

The present invention was accomplished in consideration of the above issues and the present invention aims to provide an oral pharmaceutical composition in which dose adjustment is easy, ingestibility is high and dissolution properties are optimized without using surfactants, and that is suitable especially for formulating of drugs having a narrow therapeutic range.

As a result of intensive research to achieve the above purpose, the present inventors accomplished the present invention by the findings that particulate formulations in powdery or granular form, especially particulate formulations in granular form with a particle diameter of 0.5 to 10 mm with easy dose-adjustment and high ingestibility can be prepared by a simple method which comprises incorporating an active ingredient such as drugs subject to TDM or anti-cancer drugs to a hydrophobic liquid and making an oral pharmaceutical composition wherein droplets of the hydrophobic liquid containing this active ingredient are dispersed in a composition containing a water-soluble polymer substance and an excipient, and adding dropwise, spraying or discharging the aqueous solution of the oral pharmaceutical composition in a cooling medium or in the air for shaping, and that the particulate formulations which show good dissolution properties without using surfactants and a sufficient bioavailability is obtained.

Therefore, the present invention provides a method for producing the following oral pharmaceutical compositions and the particulate formulations consisting of the compositions.
[1] An oral pharmaceutical composition not containing surfactant essentially consisting of:
   0.1 to 20 parts by mass of an active ingredient (A);
   1 to 100 parts by mass of a hydrophobic liquid (B);
   100 parts by mass of a water-soluble polymer substance (C); and
   1 to 300 parts by mass of an excipient (D), wherein the active ingredient (A) is incorporated in the hydrophobic liquid (B), and this hydrophobic liquid (B) is dispersed in a composition comprising the water-soluble polymer substance (C) and the excipient (D),

   wherein the active ingredient (A) is one or two or more usable in combination of drugs subject to Therapeutic Drug Monitoring (TDM) selected from digoxin, theophylline, procainamide, N-acetylprocainamide, aprindine, disopyramide, lidocaine, pilsicainide, propafenone, mexiletine, flecainide, quinidine, cibenzoline succinate, amiodarone, pirmenol, bepridil, phenobarbital, nitrazepam, primidone, diazepam, phenytoin, carbamazepine, zonisamide, ethosuximide, acetazolamide, clobazam, sodium valproate, trimethadione, clonazepam, sultiame, gabapentin, levetiracetam, topiramate, lamotrigine, gentamicin, amikacin, tobramycin, arbekacin, vancomycin, teicoplanin, voriconazole, cyclosporine, tacrolimus, everolimus, mycophenolate mofetil, salicylic acid, methotrexate, haloperidol, bromperidol, lithium carbonate and imatinib, or one or two or more usable in combination of an anti-cancer drug selected from cyclophosphamide, ifosfamide, melphalan, busulfan, thiotepa, nimustine, ranimustine, dacarbazine, procarbazine, temozolomide, carmustine, streptozocin, bendamustine, cisplatin, carboplatin, oxaliplatin, nedaplatin, fluorouracil, cytarabine, gemcitabine, irinotecan, nogitecan, doxorubicin, etoposide, vinblastine, vincristine, vindesine, vinorelbine, mitomycin C, doxorubicin, epirubicin, daunorubicin, bleomycin, gefitinib, erlotinib, afatinib, dasatinib, bosutinib, vandetanib, sunitinib, axitinib, pazopanib, lenvatinib, lapatinib, nintedanib, nilotinib, crizotinib, alectinib, ruxolitinib, tofacitinib, sorafenib, vemurafenib, bortezomib, sirolimus and temsirolimus, and
   wherein the hydrophobic liquid (B) is one or two or more selected from benzyl benzoate, octyl decyl triglyceride, triethyl citrate, dimethyl polysiloxane, cinnamaldehyde, triacetin, piperonyl butoxide, diethyl phthalate, dibutyl phthalate, butyl phthalyl butyl glycolate and ethyl butyrate.
[2] The oral pharmaceutical composition according [1], wherein the water-soluble polymer substance (C) is one or two or more selected from gelatin, carrageenan, xanthan gum, locust bean gum and agar.
[3] The oral pharmaceutical composition according to any one of [1] to [2], wherein the excipient (D) is one or two or more selected from isomalt, erythritol, xylitol, glycerin, sorbitol, maltitol, mannitol, lactitol, reduced palatinose, reduced sugar syrup and powdered reduced maltose syrup.
[4] An oral pharmaceutical composition not containing surfactant essentially consisting of:
   0.00001 parts by mass or more and less than 0.1 parts by mass of an active ingredient (A);
   1 to 100 parts by mass of a hydrophobic liquid (B);
   100 parts by mass of a water-soluble polymer substance (C); and
   1 to 300 parts by mass of an excipient (D), wherein the active ingredient (A) is incorporated in the hydrophobic liquid (B), and this hydrophobic liquid (B) is dispersed in a composition comprising the water-soluble polymer substance (C) and the excipient (D),
   wherein the active ingredient (A) is one or two or more usable in combination of drugs subject to Therapeutic Drug Monitoring (TDM) selected from digoxin, theophylline, procainamide, N-acetylprocainamide, aprindine, disopyramide, lidocaine, pilsicainide, propafenone, mexiletine, flecainide, quinidine, cibenzoline succinate, amiodarone, pirmenol, bepridil, phenobarbital, nitrazepam, primidone, diazepam, phenytoin, carbamazepine, zonisamide, ethosuximide, acetazolamide, clobazam, sodium valproate, trimethadione, clonazepam, sultiame, gabapentin, levetiracetam, topiramate, lamotrigine, gentamicin, amikacin, tobramycin, arbekacin, vancomycin, teicoplanin, voriconazole, cyclosporine, tacrolimus, everolimus, mycophenolate mofetil, salicylic acid, methotrexate, haloperidol, bromperidol, lithium carbonate and imatinib or one or two or more usable in combination of an anti-cancer drug selected from cyclophosphamide, ifosfamide, melphalan, busulfan, thiotepa, nimustine, ranimustine, dacarbazine, procarbazine, temozolomide, carmustine, streptozocin, bendamustine, cisplatin, carboplatin, oxaliplatin, nedaplatin, fluorouracil, cytarabine, gemcitabine, irinotecan, nogitecan, doxorubicin, etoposide, vinblastine, vincristine, vindesine, vinorelbine, mitomycin C, doxorubicin, epirubicin, daunorubicin, bleomycin, gefitinib, erlotinib, afatinib, dasatinib, bosutinib, vandetanib, sunitinib, axitinib, pazopanib, lenvatinib, lapatinib, nintedanib, nilotinib, crizotinib, alectinib, ruxolitinib, tofacitinib, sorafenib, vemurafenib, bortezomib, sirolimus and temsirolimus, and
   wherein the hydrophobic liquid (B) is one or two or more selected from benzyl benzoate, octyl decyl triglyceride, triethyl citrate, dimethyl polysiloxane, cinnamaldehyde, triacetin, piperonyl butoxide, diethyl phthalate, dibutyl phthalate, butyl phthalyl butyl glycolate and ethyl butyrate.
[5] A method for producing a particulate formulation in powdery or granular form consisting of the oral pharmaceutical composition according to any one of [1] to [3], the method comprising:
   preparing a water-soluble polymer aqueous solution by dissolving 100 parts by mass of the water-soluble polymer substance (C) and 1 to 300 parts by mass of the excipient (D) in water and preparing an active ingredient solution by dissolving or dispersing 0.1 to 20 parts by mass of the active ingredient (A) in 1 to 100 parts by mass of the hydrophobic liquid (B);
   obtaining a mixed aqueous solution where the hydrophobic liquid (B) comprising the active ingredient (A) is dispersed in said water-soluble polymer aqueous solution by mixing this active ingredient solution into the water-soluble polymer aqueous solution; and
   shaping this mixed aqueous solution into particles in powdery or granular form by adding dropwise, spraying or discharging the mixed aqueous solution in a cooling medium having no compatibility with said aqueous solution or in the air followed by drying the particles such that their water content is 1 to 20 mass%.
[6] The method for producing a particulate formulation in powdery or granular form according to [5], wherein the mixed aqueous solution is made into granules with a particle diameter of 0.5 to 10 mm by adding the mixed aqueous solution dropwise in a cooling medium having no compatibility with said aqueous solution followed by shaping.
[7] A method for producing a particulate formulation in powdery or granular form consisting of the oral pharmaceutical composition according to [4], the method comprising:
   preparing a water-soluble polymer aqueous solution by dissolving 100 parts by mass of the water-soluble polymer substance (C) and 1 to 300 parts by mass of the excipient (D) in water and preparing an active ingredient solution by dissolving or dispersing 0.00001 parts by mass or more and less than 0.1 parts by mass of the active ingredient (A) in 1 to 100 parts by mass of the hydrophobic liquid (B);
   obtaining a mixed aqueous solution where the hydrophobic liquid (B) comprising the active ingredient (A) is dispersed in said water-soluble polymer aqueous solution by mixing this active ingredient solution into the water-soluble polymer aqueous solution; and
   shaping this mixed aqueous solution into particles in powdery or granular form by adding dropwise, spraying or discharging the mixed aqueous solution in a cooling medium having no compatibility with said aqueous solution or in the air followed by drying the particles such that their water content is 1 to 20 mass%.
[8] The method for producing a particulate formulation in powdery or granular form according to [7], wherein the mixed aqueous solution is made into granules with a particle diameter of 0.5 to 10 mm by adding the mixed aqueous solution dropwise in a cooling medium having no compatibility with said aqueous solution followed by shaping.

### ADVANTAGEOUS EFFECTS OF INVENTION

According to the present invention, an oral pharmaceutical composition in which dose adjustment is easy, ingestibility is high and dissolution properties are optimized, and that is suitable especially for formulating of drugs having a narrow therapeutic range can be easily obtained by a relatively simple method. Moreover, patients taking medication can expect an improvement in medication adherence, and dispensing workplace can expect simplification or omission of dispensing procedures.

### BRIEF DESCRIPTION OF DRAWINGS

- [Fig. 1]: Fig. 1 is a micrograph showing the "mixed liquid" prepared in Example 8.
- [Fig. 2]: Fig. 2 is a micrograph showing the "mixed liquid" prepared in Example 11A.
- [Fig. 3]: Fig. 3 is a micrograph showing the "mixed liquid" prepared in Comparative Example 1A.
- [Fig. 4]: Fig. 4 is a micrograph showing the "mixed liquid" prepared in Comparative Example 7.
- [Fig. 5]: Fig. 5 is a graph showing the results of the dissolution test with a test liquid of pH 1.2.
- [Fig. 6]: Fig. 6 is a graph showing the results of the dissolution test with a test liquid of pH 5.0.
- [Fig. 7]: Fig. 7 is a graph showing the results of the dissolution test with a test liquid of pH 6.8.
- [Fig. 8]: Fig. 8 is a graph showing the results of the dissolution test with water.

### DESCRIPTION OF EMBODIMENTS

The present invention is more specifically described below.
As described above, the oral pharmaceutical composition of the present invention contains 0.1 to 20 parts by mass of an active ingredient (A), 1 to 100 parts by mass of a hydrophobic liquid (B), 100 parts by mass of a water-soluble polymer substance (C) and 1 to 300 parts by mass of an excipient (D), and contains no surfactant, and is a dispersion of the hydrophobic liquid (B) containing the above active ingredient (A) in the composition containing the above water-soluble polymer substance (C) and the excipient (D).

The above active ingredient (A) is suitably applicable especially to drugs subject to Therapeutic Drug Monitoring (TDM) and anti-cancer drugs as described above. In this case, examples of drugs subject to TDM include digoxin, theophylline, procainamide, N-acetylprocainamide, aprindine, disopyramide, lidocaine, pilsicainide, propafenone, mexiletine, flecainide, quinidine, cibenzoline succinate, amiodarone, pirmenol, bepridil, phenobarbital, nitrazepam, primidone, diazepam, phenytoin, carbamazepine, zonisamide, ethosuximide, acetazolamide, clobazam, sodium valproate, trimethadione, clonazepam, sultiame, gabapentin, levetiracetam, topiramate, lamotrigine, gentamicin, amikacin, tobramycin, arbekacin, vancomycin, teicoplanin, voriconazole, cyclosporine, tacrolimus, everolimus, mycophenolate mofetil, salicylic acid, methotrexate, haloperidol, bromperidol, lithium carbonate, imatinib and the like, and one or two or more usable in combination thereof can be used.

Moreover, examples of anti-cancer drugs include cyclophosphamide, ifosfamide, melphalan, busulfan, thiotepa, nimustine, ranimustine, dacarbazine, procarbazine, temozolomide, carmustine, streptozocin, bendamustine, cisplatin, carboplatin, oxaliplatin, nedaplatin, fluorouracil, cytarabine, gemcitabine, irinotecan, nogitecan, doxorubicin, etoposide, vinblastine, vincristine, vindesine, vinorelbine, mitomycin C, doxorubicin, epirubicin, daunorubicin, bleomycin, gefitinib, erlotinib, afatinib, dasatinib, bosutinib, vandetanib, sunitinib, axitinib, pazopanib, lenvatinib, lapatinib, nintedanib, nilotinib, crizotinib, alectinib, ruxolitinib, tofacitinib, sorafenib, vemurafenib, bortezomib, sirolimus, temsirolimus and the like, and one or two or more usable in combination thereof can be used.

It is preferable that an amount of this active ingredient (A) to be blended is 0.1 to 20 parts by mass based on 100 parts by mass of the water-soluble polymer substance (C) as described above, more preferably 0.5 to 10 parts by mass, and further preferably 1 to 5 parts by mass. It depends on kind of active ingredient (A), but when the amount of the active ingredient (A) to be blended is less than 0.1 parts by mass, obtaining a sufficient therapeutic effect by adequate dose administering becomes difficult, and it may be less usable as a pharmaceutical. On the other hand, when it exceeds 20 parts by mass, it depends on solubility of the active ingredient (A) to the hydrophobic liquid (B), but the amount of the hydrophobic liquid (B) to be blended increases and formability of the composition decreases, which may cause inconveniences such as, for example, that it may become difficult to shape the composition into a particulate form.
However, for example, there are no particular limitations, but all vitamins such as alfacalcidol, eldecalcitol, calcitriol and falecalcitriol, clenbuterol hydrochloride, desmopressin acetate hydrate, nalfurafine hydrochloride, fentanyl citrate, beraprost sodium, pergolide mesylate, misoprostol, iodine lecithin, ramosetron hydrochloride, limaprost alfadex, lubiprostone and the like may also be made into formulations of low dosage with a very low content, and in the case of such active ingredients, the amount of the active ingredient to be blended can be less than 0.1 parts by mass, concretely 0.00001 parts by mass or more and less than 0.1 parts by mass, and particularly can be 0.001 parts by mass or more and less than 0.1 parts by mass.

The above hydrophobic liquid (B) is a hydrophobic liquid that can dissolve or disperse well the above active ingredient (A) and is not a surfactant. In this case, the "hydrophobicity" of this hydrophobic liquid (B) should disperse as liquid particles containing the active ingredient (A), without dissolving into the water-based composition containing the water-soluble polymer substance (C) and the excipient (D) described below. More specifically, if it is, for example, a liquid at 20°C and its solubility in water at 20°C is 10 mass% or less, it can be used as the above hydrophobic liquid (B). When this "hydrophobicity" is insufficient, it becomes difficult to remain dispersed in the composition while containing the above active ingredient (A), and all or much of the active ingredient (A) becomes contained in the composition as crystals, which makes it impossible to achieve the purpose of the present invention.

Specifically, examples of this hydrophobic liquid (B) include propylene glycol monocaprylate, propylene glycol dicaprylate, propylene glycol dicaprate, propylene glycol monolaurate, propylene glycol monooleate, benzyl benzoate, octyl decyl triglyceride, oleic acid, triethyl citrate, dimethyl polysiloxane, cinnamaldehyde, medium chain mono-diglyceride, medium chain fatty acid triglyceride, triacetin, piperonyl butoxide, diethyl phthalate, dibutyl phthalate, butyl phthalyl butyl glycolate, octyl dodecyl myristate, ethyl butyrate and the like, and one or two or more thereof can be suitably used.

An amount of this hydrophobic liquid (B) to be blended is 1 to 100 parts by mass based on 100 parts by mass of the water-soluble polymer substance (C) as described above, preferably 1 to 50 parts by mass, and more preferably 10 to 30 parts by mass. When the amount of hydrophobic liquid (B) to be blended is less than 1 part by mass, it depends on the amount of the above active ingredient (A) to be blended, but the active ingredient cannot be contained satisfactorily in this hydrophobic liquid (B). As a result much of the active ingredient becomes present directly in the composition, and when it becomes this way, the dissolution properties of the active ingredient decrease. On the other hand, when it exceeds 100 parts by mass, the formability of the composition decreases as described above, and for example, it becomes difficult to shape the composition into a particulate form.

The above water-soluble polymer substance (C) which serves as base for the oral pharmaceutical composition of the present invention with the above excipient (D) should be able to serve this role and should be selected and be used appropriately. Specifically, there are no particular limitations, but examples include gelatin, carrageenan, xanthan gum, locust bean gum, agar and the like, and one or two or more thereof can be used.

Moreover, the above excipient (D) can be selected and used appropriately from publicly known materials, and is not particularly limited, but examples include isomalt, erythritol, xylitol, glycerin, sorbitol, maltitol, mannitol, lactitol, reduced palatinose, reduced sugar syrup, powdered reduced maltose syrup and the like, and one or two or more thereof can be used.

An amount of this excipient (D) to be blended is 1 to 300 parts by mass based on 100 parts by mass of the above water-soluble polymer substance (C), preferably 50 to 200 parts by mass, and more preferably 100 to 170 parts by mass, and should be set appropriately in this range depending on form of the oral pharmaceutical composition and type of the excipient. When the amount to be blended is less than 1 part by mass, inconveniences may occur, such as the formability decreasing, which, for example, makes satisfactorily shaping particles difficult, and low dissolution properties, which may cause not to sufficiently obtain therapeutic effect of the active ingredient (A). On the other hand, when it exceeds 300 parts by mass, fluidity when shaping the composition decreases, the preparation of the composition becomes difficult, or shaping the composition into the desired form becomes difficult depending on shaping method and dosage form.

For example, when making the oral pharmaceutical composition of the present invention into a particulate formulation in powdery or granular form, no other additives are particularly needed, and it is a particulate formulation typically consisting of the above four components (A) to (D) and water. However, it may be blended with known additives within a range not deviating from the purpose of the present invention depending on dosage form. For example, sweeteners, coloring agents, preservatives, lubricants, thickeners, stabilizers, antioxidants, bleaching agents, flavoring agents, acidulants, seasonings, pH adjusters, and all other agents for production can be blended in appropriate amounts.

Here, the oral pharmaceutical composition of the present invention is characterized in that it contains no surfactant. In this case, "containing no surfactant" in the present invention refers to
(1) when no component having a surfactant action is contained in the oral pharmaceutical composition at all, and,
(2) when, even if the oral pharmaceutical composition contains a component having a surfactant action, its amount is less than an amount significantly changing the dispersion state of the hydrophobic liquid (B) in the water-based composition containing the water-soluble polymer substance (C) and the excipient (D).

The "amount significantly changing the dispersion state of the hydrophobic liquid" in the above (2) refers, for example, to the amount which changes by 20% or more the maximum diameter of the liquid particles of the hydrophobic liquid (B) compared to when this component is not added, when observing with a microscope the maximum diameter of the liquid particles of the hydrophobic liquid (B) in the mixed aqueous solution prepared by the method described below. Namely, "containing no surfactant" means that it is acceptable that components having a surfactant action is contained in the oral pharmaceutical composition of the present invention, if it is an amount not significantly changing the dispersion state in the mixed aqueous solution of the hydrophobic liquid (B). In other words, in the present invention, even when containing components having a surfactant action, if its amount is less than the amount necessary to exhibit a substantial surfactant action, such component is not considered as a surfactant.

More specifically, there are no particular limitations, but if adding pharmaceutical additives the application of which is indicated as "surfactant" in the "Japanese Pharmaceutical Excipients Directory 2016 (Yakuji Nippo, Limited)" in effective amounts as surfactant, they fall into category of "surfactant" in the present invention. This is the case when adding in effective amounts, for example, caprylocaproyl polyoxy glyceride, cholesterol, sucrose fatty acid ester, stearyl alcohol, polyoxyl 40 stearate, cetanol, sorbitan fatty acid ester, sorbitan sesquioleate, sorbitan trioleate, polyoxyethylene hardened castor oil 60, polyoxyethylene (105) polyoxypropylene (5) glycol, polyoxyethylene (160) polyoxypropylene (30) glycol, polyoxyl 35 castor oil, polysorbate 20, polysorbate 60, polysorbate 80, macrogol 400, sorbitan monooleate, glyceryl monostearate, sorbitan monolaurate, sodium lauryl sulfate, lauromacrogol and the like.

As described above, the oral pharmaceutical composition of the present invention is prepared so that the hydrophobic liquid (B) containing the above active ingredient (A) be dispersed in the composition containing the above water-soluble polymer substance (C) and the excipient (D). There are no limitations to a preparation method of the oral pharmaceutical composition of the present invention, however for example, it is preferable to make a particulate formulation in powdery or granular form by the following method. And according to this method, the particulate formulation in granular form with a particle diameter of 0.5 to 10 mm, in which the dose adjustment is easy and the ingestibility is high, and that is suitable for drugs subject to TDM and anti-cancer drugs, can be produced easily.

Namely, first, a water-soluble polymer aqueous solution is prepared by dissolving 100 parts by mass of the above water-soluble polymer substance (C) and 1 to 300 parts by mass of the excipient (D) in water and an active ingredient solution is prepared by dissolving or dispersing 0.1 to 20 parts by mass of the active ingredient (A) in 1 to 100 parts by mass of the above hydrophobic liquid (B). Then, this active ingredient solution is mixed with the above water-soluble polymer aqueous solution, and a mixed aqueous solution where the above hydrophobic liquid (B) containing the above active ingredient (A) is dispersed in the water-soluble polymer aqueous solution is obtained. This mixed aqueous solution is shaped into particles in powdery or granular form by adding dropwise, spraying or discharging the mixed aqueous solution in a cooling medium having no compatibility with the aqueous solution or in the air, and then dried shaped particles thus obtained such that their water content becomes 1 to 20 mass%. This method allows a particulate formulation in powdery or granular form consisting of the above oral pharmaceutical composition of the present invention to be obtained. However, if making it a low dosage formulation as described above, the amount of the above active ingredient (A) to be blended can be 0.00001 parts by mass or more and less than 0.1 parts by mass.

In this production method, by adopting a shaping method that adds dropwise the above mixed aqueous solution in the cooling medium having no compatibility with the aqueous solution, the mixed aqueous solution can easily be made into granules with a particle diameter of 0.5 to 10 mm, preferably 1 to 5 mm and more preferably 2 to 4 mm. As a result, the particulate formulation in which the dose adjustment is easy and the ingestibility is high, and that is suitable for drugs subject to TDM and anti-cancer drugs can easily be obtained.

### EXAMPLES

The present invention is shown more specifically below, by showing Examples and Comparative Examples, but the present invention is not limited to the following examples.

### [Example 1]

A "gelatin aqueous solution" (water-soluble polymer aqueous solution) was obtained by adding 2.496 g of gelatin and 2.496 g of lactitol to 7.738 g of purified water, then by heating an obtained mixture at 55 to 65°C to dissolve the gelatin and the lactitol. On the other hand, an "active ingredient solution" was obtained by adding 0.022 g of sirolimus to 2.247 g of diethyl phthalate, then heating an obtained mixture at 55 to 65°C to dissolve the sirolimus. A "mixed liquid" (mixed aqueous solution) was obtained by adding gradually the "active ingredient solution" into the "gelatin aqueous solution" while stirring the "gelatin aqueous solution" at 55 to 65°C. In this "mixed liquid," the droplets of diethyl phthalate where sirolimus was completely dissolved were uniformly dispersed in the "gelatin aqueous solution."

The above "mixed liquid" was added dropwise by 25 to 40 mg at a time in 250 mL of medium chain fatty acid triglyceride which was cooled to 10 to 15°C, then solidified into granules and recovered after 5 min. The total mass of the recovered undried particles was 13.5 g. A particulate formulation of an oral pharmaceutical composition was obtained by air drying these undried particles at 10 to 20°C (relative humidity 20 to 40%) for 16 h. There, the total mass decreased by 5.21 g, which corresponds to 38.6 mass%, due to the above drying treatment, to become 8.29 g. The loss on drying of this particulate formulation was 12.0%, the mass per particle was 20.1 mg on average (water content: about 13.0 mass%), and the particle diameter was 2.6 to 3.3 mm.

### [Example 2]

0.011 g of xanthan gum and 0.011 g of locust bean gum were added to 8.672 g of purified water, then heated at 75 to 80°C to dissolve the xanthan gum and the locust bean gum, and an obtained mixture was allowed to cool at room temperature for 2 h. The "gelatin aqueous solution" was obtained by adding 2.168 g of gelatin and 2.168 g of lactitol to this aqueous solution, then heating an obtained mixture at 55 to 65°C to dissolve the gelatin and the lactitol. On the other hand, the "active ingredient solution" was obtained by adding 0.020 g of sirolimus to 1.951 g of propylene glycol monocaprylate, then heating an obtained mixture at 55 to 65°C to dissolve the sirolimus. The "mixed liquid" was obtained by adding gradually the "active ingredient solution" into the "gelatin aqueous solution" while stirring the "gelatin aqueous solution" at 55 to 65°C. In this "mixed liquid," the droplets of propylene glycol monocaprylate where sirolimus was completely dissolved were uniformly dispersed in the "gelatin aqueous solution."

This "mixed liquid" was added dropwise by 25 to 40 mg at a time in 250 mL of medium chain fatty acid triglyceride which was cooled to 10 to 15°C, then solidified and recovered after 5 min. The total mass of the recovered undried particles was 13.2 g. A particulate formulation of an oral pharmaceutical composition was obtained by air drying these undried particles at 10 to 20°C (relative humidity20 to 40%) for 16 h. There, the total mass decreased by 5.98 g, which corresponds to 45.3 mass%, due to the above drying treatment, to become 7.22 g. The loss on drying of this particulate formulation was 11.7%, the mass per particle was 19.5 mg on average (water content: about 12.5 mass%) and the particle diameter was 2.5 to 3.1 mm.

### [Example 3]

7.67 g of a particulate formulation was obtained by preparation in the same way as in Example 1, except that in the "gelatin aqueous solution" of Example 1, 7.181 g of purified water, 2.688 g of gelatin and 3.840 g of reduced sugar syrup were used, and in the "active ingredient solution", 0.042 g of carbamazepine and 1.250 g of diethyl phthalate were used. The loss on drying of this particulate formulation was 10.5%, the mass per particle was 21.3 mg on average (water content: about 11.2 mass%), and the particle diameter was 2.8 to 3.4 mm.

### [Example 4]

7.83 g of a particulate formulation was obtained by preparation in the same way as in Example 3, except that in the "active ingredient solution" of Example 3, triethyl citrate was used instead of diethyl phthalate. The loss on drying of this particulate formulation was 10.8%, the mass per particle was 21.0 mg on average (water content: about 11.5 mass%), and the particle diameter was 2.9 to 3.4 mm.

### [Example 5]

7.45 g of a particulate formulation was obtained by preparation in the same way as in Example 3, except that in the "active ingredient solution" of Example 3, oleic acid was used instead of diethyl phthalate. The loss on drying of this particulate formulation was 10.0%, the mass per particle was 20.3 mg on average (water content: about 10.8 mass%), and the particle diameter was 2.8 to 3.3 mm.

### [Example 6]

8.41 g of a particulate formulation was obtained by preparation in the same way as in Example 1, except that in the "gelatin aqueous solution" of Example 1, 7.381 g of purified water, 2.381 g of gelatin and 3.968 g of lactitol were used, and in the "active ingredient solution", 0.423 g of cyclosporine and 0.847 g of propylene glycol monocaprylate were used. The loss on drying of this particulate formulation was 12.9%, the mass per particle was 23.9 mg on average (water content: about 12.9 mass%), and the particle diameter was 2.9 to 3.5 mm.

### [Example 7]

8.13 g of a particulate formulation was obtained by preparation in the same way as in Example 1, except that in the "gelatin aqueous solution" of Example 1, 7.254 g of purified water, 2.340 g of gelatin and 3.900 g of lactitol were used, and in the "active ingredient solution", 0.430 g of cyclosporine and 1.076 g of oleic acid were used. The loss on drying of this particulate formulation was 11.1%, the mass per particle was 22.8 mg on average (water content: about 11.9 mass%), and the particle diameter was 2.8 to 3.4 mm.

### [Example 8]

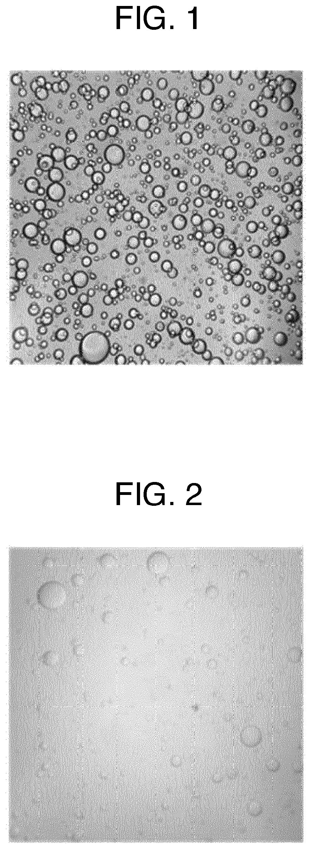

8.38 g of a particulate formulation was obtained by preparation in the same way as in Example 1, except that in the "gelatin aqueous solution" of Example 1, 7.122 g of purified water, 2.298 g of gelatin and 3.829 g of lactitol were used, and in the "active ingredient solution", 0.438 g of cyclosporine and 1.313 g of triethyl citrate were used. The loss on drying of this particulate formulation was 11.1%, the mass per particle was 22.8 mg on average (water content: about 12.5 mass%) and the particle diameter was 2.8 to 3.4 mm. The micrograph of the above "mixed liquid" in the preparation of this example is shown in Fig. 1. As shown in Fig. 1, it was confirmed that it was a "mixed liquid," wherein the droplets of triethyl citrate where cyclosporine was completely dissolved were uniformly dispersed in the "gelatin aqueous solution."

### [Example 9]

7.54 g of a particulate formulation was obtained by preparation in the same way as in Example 1, except that in the "gelatin aqueous solution" of Example 1, 7.529 g of purified water, 2.818 g of gelatin and 4.026 g of reduced sugar syrup were used, and in the "active ingredient solution", 0.039 g of everolimus and 0.588 g of medium chain fatty acid triglyceride were used. The loss on drying of this particulate formulation was 10.0%, the mass per particle was 19.8 mg on average (water content: about 11.1 mass%), and the particle diameter was 2.8 to 3.3 mm.

### [Example 10]

7.31 g of a particulate formulation was obtained by preparation in the same way as in Example 9, except that in the "active ingredient solution" of Example 9, piperonyl butoxide was used instead of medium chain fatty acid triglyceride. The loss on drying of this particulate formulation was 9.70%, the mass per particle was 19.1 mg on average (water content: about 10.5 mass%), and the particle diameter was 2.8 to 3.2 mm.

### [Example 11A]

7.42 g of a particulate formulation was obtained by preparation in the same way as in Example 1, except that in the "gelatin aqueous solution" of Example 1, 8.757 g of purified water, 2.825 g of gelatin and 2.825 g of powdered reduced maltose syrup were used, and in the "active ingredient solution", 0.039 g of tacrolimus hydrate and 0.555 g of triethyl citrate were used. The loss on drying of this particulate formulation was 9.40%, the mass per particle was 18.9 mg on average (water content: about 10.3 mass%) and the particle diameter was 2.9 to 3.2 mm. The micrograph of the "mixed liquid" in the preparation of this example is shown in Fig. 2. As shown in Fig. 2, it was confirmed that it was a "mixed liquid," wherein the droplets of triethyl citrate where tacrolimus hydrate was completely dissolved were uniformly dispersed in the "gelatin aqueous solution."

### [Example 11B]

7.21 g of a particulate formulation was obtained by preparation in the same way as in Example 11A, except that in the "gelatin aqueous solution" of Example 11A, 8.280 g of purified water, 2.671 g of gelatin and 2.671 g of powdered reduced maltose syrup were used, and in the "active ingredient solution", 0.042 g of tacrolimus hydrate and 1.336 g of triethyl citrate were used. The loss on drying of this particulate formulation was 9.00%, the mass per particle was 19.2 mg on average (water content: about 9.80 mass%), and the particle diameter was 2.9 to 3.2 mm.

### [Example 12]

7.37 g of a particulate formulation was obtained by preparation in the same way as in Example 11A, except that in the "active ingredient solution" of Example 11A, triacetin was used instead of triethyl citrate. The loss on drying of this particulate formulation was 9.50%, the mass per particle was 19.3 mg on average (water content: about 10.7 mass%), and the particle diameter was 2.9 to 3.3 mm.

### [Example 13]

7.27 g of a particulate formulation was obtained by preparation in the same way as in Example 11A, except that in the "active ingredient solution" of Example 11A, butyl phthalyl butyl glycolate was used instead of triethyl citrate. The loss on drying of this particulate formulation was 10.3%, the mass per particle was 19.9 mg on average (water content: about 11.1 mass%), and the particle diameter was 2.8 to 3.3 mm.

### [Comparative Example 1A]

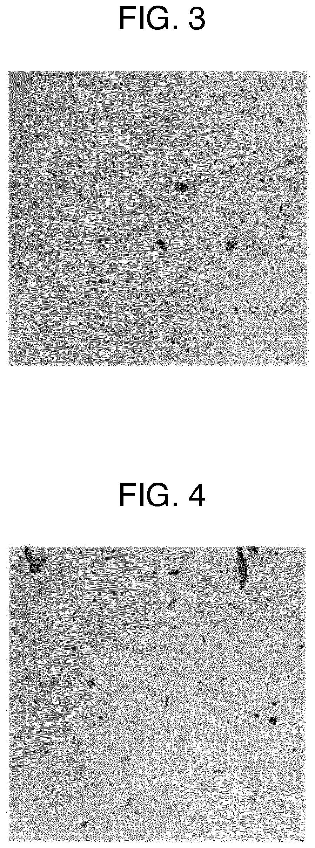

The "gelatin aqueous solution" was obtained by adding 2.934 g of gelatin and 2.934 g of powdered reduced maltose syrup to 9.095 g of purified water, then by heating an obtained mixture at 55 to 65°C to dissolve the gelatin and the powdered reduced maltose syrup. The "mixed liquid" was obtained by adding gradually 0.037 g of tacrolimus hydrate into the "gelatin aqueous solution" while stirring this "gelatin aqueous solution" at 55 to 65°C. The micrograph of this "mixed liquid" is shown in Fig. 3. As shown in Fig. 3, it was confirmed that in this "mixed liquid" tacrolimus hydrate crystals which were not dissolved were dispersed in the "gelatin aqueous solution."

This "mixed liquid" was added dropwise by 25 to 40 mg at a time in 250 mL of medium chain fatty acid triglyceride which was cooled to 10 to 15°C, then solidified and recovered after 5 min. The total mass of the recovered undried particles was 13.4 g. A particulate formulation of an oral pharmaceutical composition was obtained by air drying these undried particles at 10 to 20°C (relative humidity 20 to 40%) for 16 h. There, the total mass decreased by 6.58 g, which corresponds to 49.1 mass%, due to the above drying treatment, to become 6.82 g. The loss on drying of this particulate formulation was 10.2%, the mass per particle was 18.9 mg on average (water content: about 11.5 mass%), and the particle diameter was 2.8 to 3.2 mm.

### [Comparative Example 1B]

The "gelatin aqueous solution" was obtained by adding 2.929 g of gelatin and 2.929 g of powdered reduced maltose syrup to 9.081 g of purified water, then by heating an obtained mixture at 55 to 65°C to dissolve the gelatin and the powdered reduced maltose syrup. On the other hand, 0.037 g of tacrolimus hydrate was added to 0.023 g of triethyl citrate (corresponding to about 0.8 parts by mass of triethyl citrate based on 100 parts by mass of the above gelatin), then it was heated at 55 to 65°C for 30 min, but undissolved tacrolimus hydrate was found. A "mixed liquid" was obtained by adding the dispersion liquid of the tacrolimus hydrate and triethyl citrate into the "gelatin aqueous solution" while stirring the "gelatin aqueous solution" at 55 to 65°C. When observing this "mixed liquid" with a microscope, it was found that both the droplets of triethyl citrate where tacrolimus hydrate was dissolved and the tacrolimus hydrate crystals were dispersed in the "gelatin aqueous solution."

This "mixed liquid" was added dropwise by 25 to 40 mg at a time in 250 mL of medium chain fatty acid triglyceride which was cooled to 10 to 15°C, then solidified and recovered after 5 min. The total mass of the recovered undried particles was 13.5 g. A particulate formulation of an oral pharmaceutical composition was obtained by air drying these undried particles at 10 to 20°C (relative humidity20 to 40%) for 16 h. There, the total mass decreased by 6.66 g, which corresponds to 49.3 mass%, due to the above drying treatment, to become 6.84 g. The loss on drying of this particulate formulation was 10.0%, the mass per particle was 19.2 mg on average (water content: about 11.2 mass%) and the particle diameter was 2.7 to 3.2 mm.

### [Comparative Example 1C]

The "gelatin aqueous solution" was obtained by adding 2.336 g of gelatin and 2.336 g of powdered reduced maltose syrup to 7.242 g of purified water, then by heating an obtained mixture at 55 to 65°Cto dissolve the gelatin and the powdered reduced maltose syrup. On the other hand, the "active ingredient solution" was obtained by adding 0.048 g of tacrolimus hydrate to 3.037 g of triethyl citrate (corresponding to about 130 parts by mass of triethyl citrate based on 100 parts by mass of the above gelatin), then by heating an obtained mixture at 55 to 65°C to dissolve the tacrolimus hydrate. A "mixed liquid" was obtained by adding gradually the "active ingredient solution" into the "gelatin aqueous solution" while stirring the "gelatin aqueous solution" at 55 to 65°C. In this "mixed liquid," the droplets of triethyl citrate where tacrolimus hydrate was completely dissolved were uniformly dispersed in the "gelatin aqueous solution."

This "mixed liquid" was added dropwise by 25 to 40 mg at a time in 250 mL of medium chain fatty acid triglyceride which was cooled to 10 to 15°C, then recovered after 60 min, but the particulate formulation could not be obtained since it was not solidified.

### [Comparative Example 2]

The "gelatin aqueous solution" was obtained by adding 2.825 g of gelatin and 2.825 g of powdered reduced maltose syrup to 8.757 g of purified water, then by heating an obtained mixture at 55 to 65°C to dissolve the gelatin and the powdered reduced maltose syrup. On the other hand, the "active ingredient solution" was obtained by adding 0.039 g of tacrolimus hydrate to 0.555 g of polyoxyl 35 castor oil (surfactant), then by heating an obtained mixture at 55 to 65°C to dissolve the tacrolimus hydrate. The "mixed liquid" was obtained by adding gradually the "active ingredient solution" into the "gelatin aqueous solution" while stirring the "gelatin aqueous solution" at 55 to 65°C. In this "mixed liquid," the droplets of polyoxyl 35 castor oil where tacrolimus hydrate was completely dissolved were uniformly dispersed in the "gelatin aqueous solution."

This "mixed liquid" was added dropwise by 25 to 40 mg at a time in 250 mL of medium chain fatty acid triglyceride which was cooled to 10 to 15°C, but a particulate formulation could not be obtained since the mixed liquid blended with the medium chain fatty acid triglycerides was not solidified as spheres .

### [Comparative Example 3]

The "gelatin aqueous solution" was obtained by adding 2.825 g of gelatin and 2.825 g of powdered reduced maltose syrup to 8.757 g of purified water, then by heating an obtained mixture at 55 to 65°C to dissolve the gelatin and the powdered reduced maltose syrup. On the other hand, the "active ingredient solution" was obtained by adding 0.039 g of tacrolimus hydrate to 0.555 g of polyoxyethylene hardened castor oil 60 (surfactant), then by heating an obtained mixture at 55 to 65°C to dissolve the tacrolimus hydrate. When gradually adding the "active ingredient solution" into the "gelatin aqueous solution" while stirring the "gelatin aqueous solution" at 55 to 65°C, precipitation of tacrolimus hydrate crystals was confirmed in the "mixed liquid." Moreover, this "mixed liquid" was added dropwise by 25 to 40 mg at a time in 250 mL of medium chain fatty acid triglyceride which was cooled to 10 to 15°C, but a particulate formulation could not be obtained since the mixed liquid blended with the medium chain fatty acid triglycerides was not solidified as spheres.

### [Comparative Example 4]

It was prepared in the same way as in Comparative Example 3, except that polysorbate 80 (surfactant) was used instead of polyoxyethylene hardened castor oil 60 in the "active ingredient solution" of Comparative Example 3. Precipitation of tacrolimus hydrate crystals were confirmed in the "mixed liquid" as in Comparative Example 3, and a particulate formulation could not be obtained since the mixed liquid blended with the medium chain fatty acid triglycerides was not solidified as spheres.

### [Comparative Example 5]

It was prepared in the same way as in Comparative Example 3, except that caprylocaproyl polyoxy glyceride (surfactant) was used instead of polyoxyethylene hardened castor oil 60 in the "active ingredient solution" of Comparative Example 3. Precipitation of tacrolimus hydrate crystals were confirmed in the "mixed liquid" as in Comparative Example 3, and a particulate formulation could not be obtained since the mixed liquid blended with the medium chain fatty acid triglycerides was not solidified as spheres.

### [Comparative Example 6]

The "gelatin aqueous solution" was obtained by adding 2.825 g of gelatin and 2.825 g of powdered reduced maltose syrup to 8.757 g of purified water, then by heating an obtained mixture at 55 to 65°C to dissolve the gelatin and the powdered reduced maltose syrup. On the other hand, the "active ingredient solution" was obtained by adding 0.039 g of tacrolimus hydrate to 0.555 g of propylene glycol (solubility in water at 20°C: the amount exceeding 10 mass% is uniformly dissolved), then by heating an obtained mixture at 55 to 65°Cto dissolve the tacrolimus hydrate. When gradually adding the "active ingredient solution" into the "gelatin aqueous solution" while stirring the "gelatin aqueous solution" at 55 to 65°C, precipitation of tacrolimus hydrate crystals was confirmed in the "mixed liquid."

This "mixed liquid" was added dropwise by 25 to 40 mg at a time in 250 mL of medium chain fatty acid triglyceride which was cooled to 10 to 15°C, then solidified and recovered after 5 min. The total mass of the recovered undried particles was 13.8 g. A particulate formulation of an oral pharmaceutical composition was obtained by air drying these undried particles at 10 to 20°C (relative humidity 20 to 40%) for 16 h. There, the total mass decreased by 6.27 g, which corresponds to 45.4 mass%, due to the above drying treatment, to become 7.53 g. The loss on drying of this particulate formulation was 11.5%, the mass per particle was 20.8 mg on average (water content: about 13.0 mass%) and the particle diameter was 2.6 to 3.3 mm.

### [Comparative Example 7]

It was prepared in the same way as in Comparative Example 6, except that in the "active ingredient solution" of Comparative Example 6, macrogol 400 (surfactant) was used instead of propylene glycol. The loss on drying of the obtained particulate formulation was 11.3%, the mass per particle was 21.0 mg on average (water content: about 12.4 mass%) and the particle diameter was 2.7 to 3.2 mm. The micrograph of the "mixed liquid" in the preparation of this example is shown in Fig. 4. As shown in Fig. 4, a precipitation of tacrolimus hydrate crystals was confirmed in the "mixed liquid" as in Comparative Example 6.

As described above in each Examples and Comparative Examples, the state of the active ingredient, the presence or absence of precipitation and the formability in the production of each example were evaluated according to the following method. The results are shown in Tables 1 and 2. Moreover, the dissolution properties were evaluated by performing a dissolution test according to the following method regarding the particulate formulations of the oral pharmaceutical compositions obtained in each example. The results are shown in Tables 1 and 2.

### [State of the active ingredient]

In all the above Examples and Comparative Examples, the state of the active ingredient was evaluated by adding and stirring the hydrophobic liquid in the active ingredient during the step of preparing the "active ingredient solution," and heating an obtained mixture at 55 to 65°C for 30 min, then observing visually if the active ingredient had been dissolved. The results are shown in Tables 1 and 2. The meaning of the evaluation symbols in the tables is as follows.
∘: Dissolved.
×: Insoluble matter was found.
-: The active ingredient solution was not prepared.

### [Presence or absence of precipitation]

In all the above Examples and Comparative Examples, it was observed by microscope whether there was a precipitation of active ingredient regarding the "mixed liquid." The results are shown in Tables 1 and 2. The meaning of the evaluation symbols in the tables is as follows.
∘: No precipitation of the active ingredient was confirmed.
×: A precipitation of the active ingredient was confirmed.

### [Formability]

In all the above Examples and Comparative Examples, it was determined whether the particulate formulation was shapable from the "mixed liquid" in the preparation of the particulate formulation. The results are shown in Tables 1 and 2. The meaning of the evaluation symbols in the tables is as follows.
∘: It was shapable.
×: It could not be shaped.

### [Dissolution properties]

Regarding the particulate formulations obtained in the above Examples 1 to 13, Comparative Examples 1A and 1B, and Comparative Examples 6 and 7, a dissolution properties test was performed under the following conditions depending on an active ingredient, and it was observed visually whether the formulation and the active ingredient in the vessel remained within a predetermined time period. The results are shown in Tables 1 and 2. The meaning of the evaluation symbols in the tables is as follows.
∘: No residue of the particulate formulation and the active ingredient was found.
×: A residue of the particulate formulation and the active ingredient was found.
-: The dissolution properties test could not be performed since the particulate formulation could not be prepared.

### <Test conditions>

### (Examples 1 and 2 (component: sirolimus))

Test method: Dissolution test method 2 (Paddle method)
Test liquid: Aqueous solution of sodium lauryl sulfate 0.4%, 500 mL, 37°C
Number of revolutions: 100 rpm
Input amount: 1 mg (as sirolimus)
Evaluation time: 60 min

### (Examples 3 to 5 (component: carbamazepine))

Test method: Dissolution test method 2 (Paddle method)
Test liquid: Water, 900 mL, 37°C
Number of revolutions: 75 rpm
Input amount: 200 mg (as carbamazepine)
Evaluation time: 45 min

### (Examples 6 to 8 (component: cyclosporine))

Test method: Dissolution test method 2 (Paddle method)
Test liquid: Water, 900 mL, 37°C
Number of revolutions: 50 rpm
Input amount: 50 mg (as cyclosporine)
Evaluation time: 45 min

### (Examples 9 to 10 (component: everolimus))

Test method: Dissolution test method 2 (Paddle method)
Test liquid: Water, 900 mL, 37°C
Number of revolutions: 50 rpm
Input amount: 10 mg (as everolimus)
Evaluation time: 60 min

### (Examples 11A to 13, Comparative Examples 1A and 1B, and Comparative Examples 6 and 7 (component: tacrolimus hydrate))

Test method: Dissolution test method 2 (Paddle method)
Test liquid: Water, 900 mL, 37°C
Number of revolutions: 100 rpm
Input amount: 5 mg (as tacrolimus)
Evaluation time: 45 min

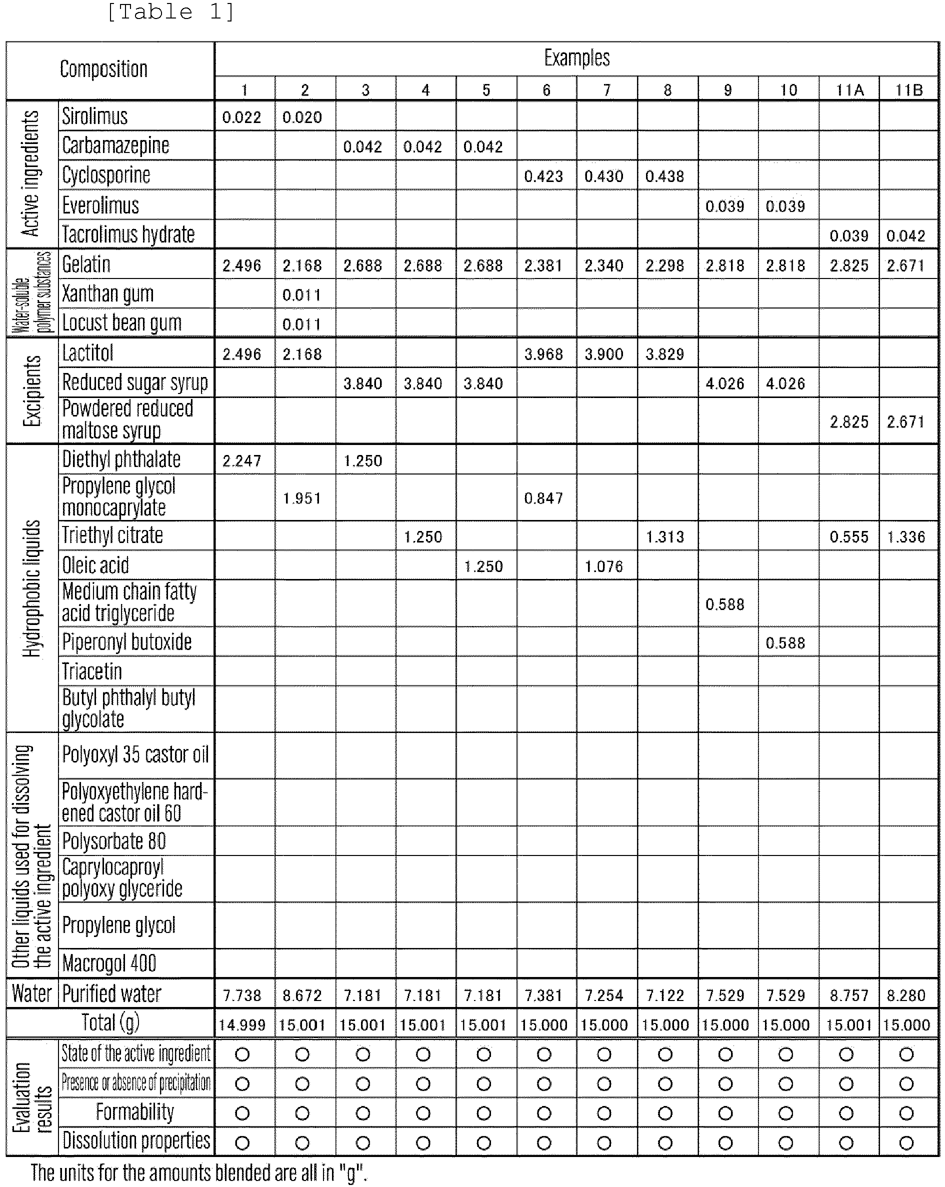

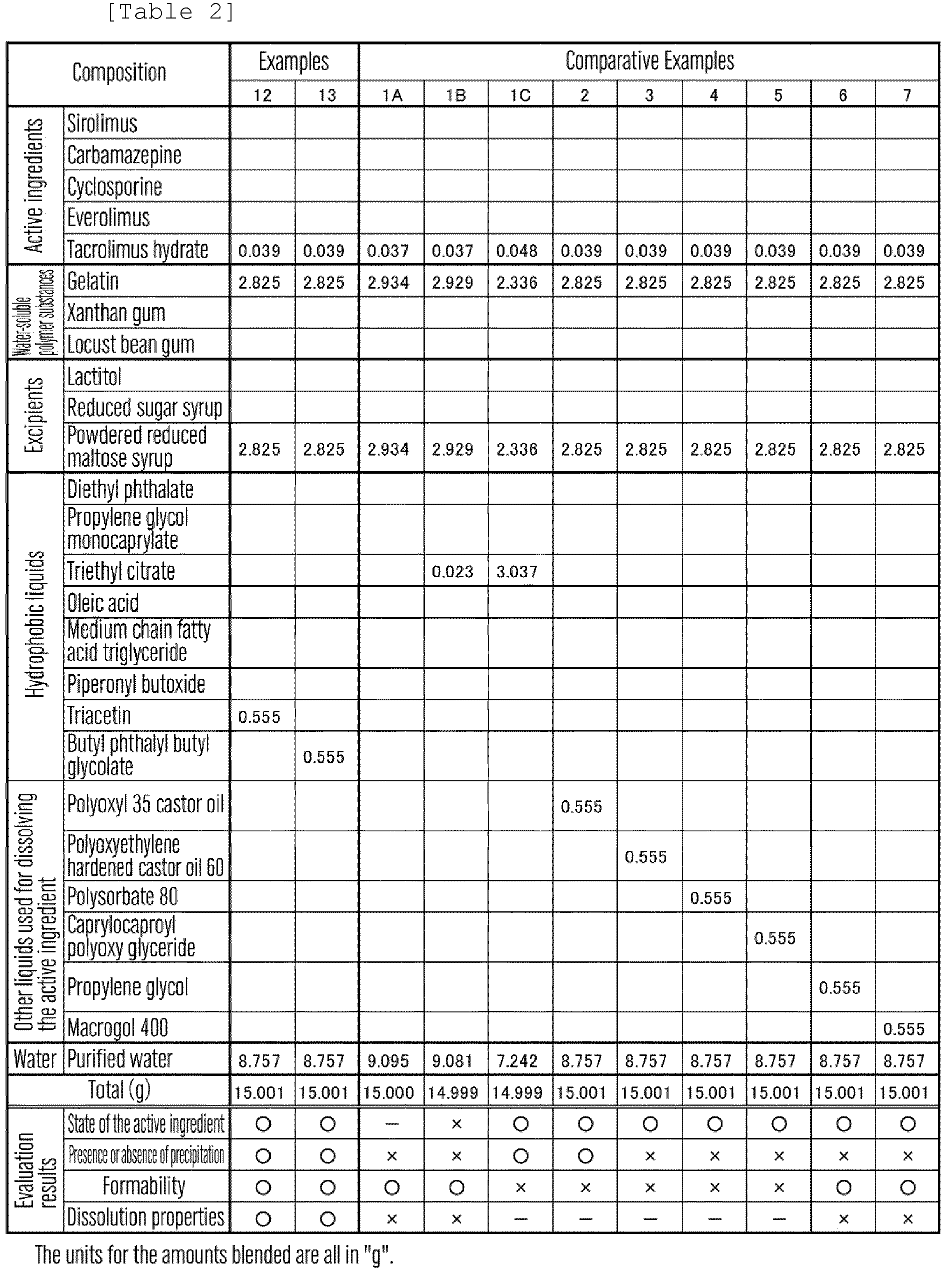

As shown in the above Tables 1 and 2, according to the oral pharmaceutical composition of the present invention, particulate formulations having 0.5 to 10 mm particle diameter can be easily obtained, and the obtained particulate formulations of the oral pharmaceutical composition was confirmed to have excellent dissolution properties of the active ingredient.

### [Dissolution test of the particulate formulation blended with tacrolimus hydrate]

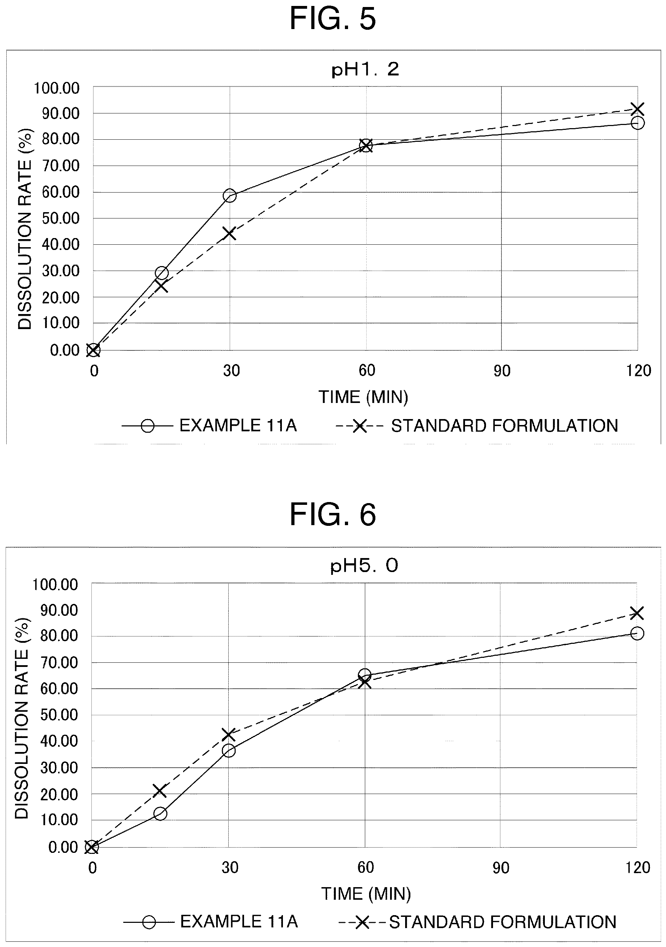

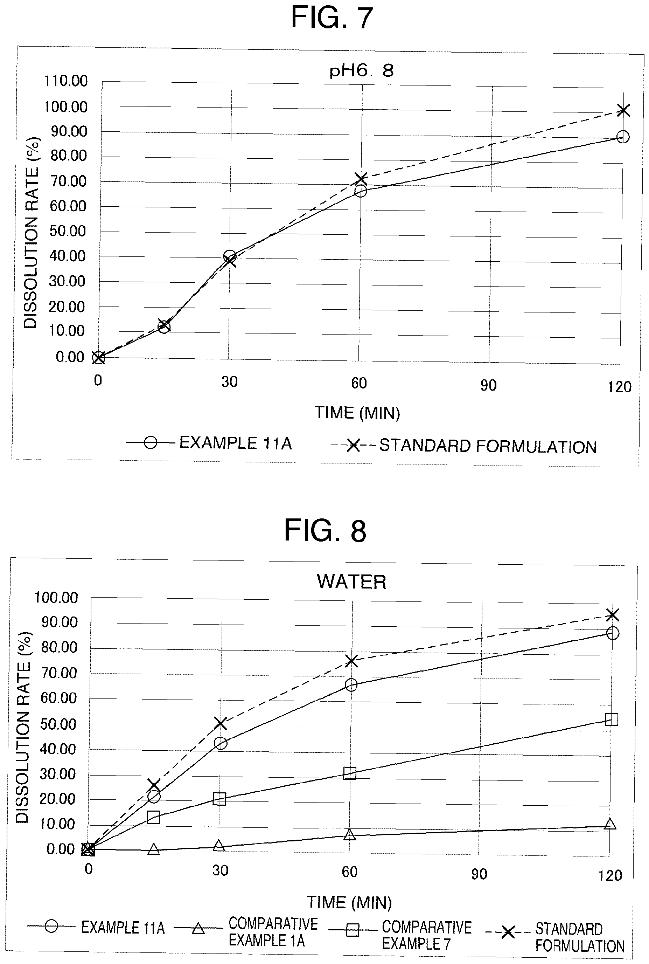

A dissolution test was performed under the following conditions regarding each particulate formulation of the above Example 11A, Comparative Example 1A and Comparative Example 7 blended with tacrolimus hydrate as the active ingredient and a standard formulation (Astellas Pharma Inc.'s "Prograf Capsules 5 mg"). The results are shown in Figs. 5 to 8. For Comparative Example 1A and Comparative Example 7, the dissolution test was performed only with water.

### <Test conditions>

Test liquid: 900 mL, 37°C for each test liquid
   (1) pH 1.2 (Japanese Pharmacopoeia Dissolution test liquid 1)
   (2) pH 5.0 (diluted Mcllvaine buffer solution)
   (3) pH 6.8 (Japanese Pharmacopoeia Dissolution test liquid 2)
   (4) Water
Number of revolutions: 50 rpm
Input amount: 5 mg (as tacrolimus)
Test method: After starting the dissolution test under the above conditions according to the Dissolution test method 2 (Paddle method), the test liquid was collected at 15, 30, 60 and 120 min, and filtered with a membrane filter having 0.45 um pore size. Excluding the first filtrate of 1 mL, the next filtrate of 1 mL was collected and this was diluted twice with ethanol, to obtain the sample solution.

### <Analysis conditions>

High-speed liquid chromatograph: Shimadzu Prominence
Detector: UV absorption photometer (measurement wavelength: 220 nm)
Column: YMC-Triart C18 (5 um) 150x4.6 mm I.D.
Column temperature: 50°C
Mobile phase: Water/2-propanol/tetrahydrofuran mixture (5:2:2)
Flow rate: 0.725 mL/min
Injection amount: 40 µL

As shown in Figs. 5 to 8, the particulate formulation of tacrolimus consisting of the oral pharmaceutical composition of the present invention was confirmed to have excellent dissolution properties that are not inferior to the standard formulation.

## Claims

1. An oral pharmaceutical composition not containing surfactant essentially consisting of:
0.1 to 20 parts by mass of an active ingredient (A);
1 to 100 parts by mass of a hydrophobic liquid (B);
100 parts by mass of a water-soluble polymer substance (C); and
1 to 300 parts by mass of an excipient (D), wherein the active ingredient (A) is incorporated in the hydrophobic liquid (B), and this hydrophobic liquid (B) is dispersed in a composition comprising the water-soluble polymer substance (C) and the excipient (D),
wherein the active ingredient (A) is one or two or more usable in combination of drugs subject to Therapeutic Drug Monitoring (TDM) selected from digoxin, theophylline, procainamide, N-acetylprocainamide, aprindine, disopyramide, lidocaine, pilsicainide, propafenone, mexiletine, flecainide, quinidine, cibenzoline succinate, amiodarone, pirmenol, bepridil, phenobarbital, nitrazepam, primidone, diazepam, phenytoin, carbamazepine, zonisamide, ethosuximide, acetazolamide, clobazam, sodium valproate, trimethadione, clonazepam, sultiame, gabapentin, levetiracetam, topiramate, lamotrigine, gentamicin, amikacin, tobramycin, arbekacin, vancomycin, teicoplanin, voriconazole, cyclosporine, tacrolimus, everolimus, mycophenolate mofetil, salicylic acid, methotrexate, haloperidol, bromperidol, lithium carbonate and imatinib, or one or two or more usable in combination of an anti-cancer drug selected from cyclophosphamide, ifosfamide, melphalan, busulfan, thiotepa, nimustine, ranimustine, dacarbazine, procarbazine, temozolomide, carmustine, streptozocin, bendamustine, cisplatin, carboplatin, oxaliplatin, nedaplatin, fluorouracil, cytarabine, gemcitabine, irinotecan, nogitecan, doxorubicin, etoposide, vinblastine, vincristine, vindesine, vinorelbine, mitomycin C, doxorubicin, epirubicin, daunorubicin, bleomycin, gefitinib, erlotinib, afatinib, dasatinib, bosutinib, vandetanib, sunitinib, axitinib, pazopanib, lenvatinib, lapatinib, nintedanib, nilotinib, crizotinib, alectinib, ruxolitinib, tofacitinib, sorafenib, vemurafenib, bortezomib, sirolimus and temsirolimus, and
wherein the hydrophobic liquid (B) is one or two or more selected from benzyl benzoate, octyl decyl triglyceride, triethyl citrate, dimethyl polysiloxane, cinnamaldehyde, triacetin, piperonyl butoxide, diethyl phthalate, dibutyl phthalate, butyl phthalyl butyl glycolate and ethyl butyrate.

2. The oral pharmaceutical composition according claim 1, wherein the water-soluble polymer substance (C) is one or two or more selected from gelatin, carrageenan, xanthan gum, locust bean gum and agar.

3. The oral pharmaceutical composition according to any one of claims 1 to 2, wherein the excipient (D) is one or two or more selected from isomalt, erythritol, xylitol, glycerin, sorbitol, maltitol, mannitol, lactitol, reduced palatinose, reduced sugar syrup and powdered reduced maltose syrup.

4. An oral pharmaceutical composition not containing surfactant essentially consisting of:
0.00001 parts by mass or more and less than 0.1 parts by mass of an active ingredient (A);
1 to 100 parts by mass of a hydrophobic liquid (B);
100 parts by mass of a water-soluble polymer substance (C); and
1 to 300 parts by mass of an excipient (D), wherein the active ingredient (A) is incorporated in the hydrophobic liquid (B), and this hydrophobic liquid (B) is dispersed in a composition comprising the water-soluble polymer substance (C) and the excipient (D),
wherein the active ingredient (A) is one or two or more usable in combination of drugs subject to Therapeutic Drug Monitoring (TDM) selected from digoxin, theophylline, procainamide, N-acetylprocainamide, aprindine, disopyramide, lidocaine, pilsicainide, propafenone, mexiletine, flecainide, quinidine, cibenzoline succinate, amiodarone, pirmenol, bepridil, phenobarbital, nitrazepam, primidone, diazepam, phenytoin, carbamazepine, zonisamide, ethosuximide, acetazolamide, clobazam, sodium valproate, trimethadione, clonazepam, sultiame, gabapentin, levetiracetam, topiramate, lamotrigine, gentamicin, amikacin, tobramycin, arbekacin, vancomycin, teicoplanin, voriconazole, cyclosporine, tacrolimus, everolimus, mycophenolate mofetil, salicylic acid, methotrexate, haloperidol, bromperidol, lithium carbonate and imatinib or one or two or more usable in combination of an anti-cancer drug selected from cyclophosphamide, ifosfamide, melphalan, busulfan, thiotepa, nimustine, ranimustine, dacarbazine, procarbazine, temozolomide, carmustine, streptozocin, bendamustine, cisplatin, carboplatin, oxaliplatin, nedaplatin, fluorouracil, cytarabine, gemcitabine, irinotecan, nogitecan, doxorubicin, etoposide, vinblastine, vincristine, vindesine, vinorelbine, mitomycin C, doxorubicin, epirubicin, daunorubicin, bleomycin, gefitinib, erlotinib, afatinib, dasatinib, bosutinib, vandetanib, sunitinib, axitinib, pazopanib, lenvatinib, lapatinib, nintedanib, nilotinib, crizotinib, alectinib, ruxolitinib, tofacitinib, sorafenib, vemurafenib, bortezomib, sirolimus and temsirolimus, and
wherein the hydrophobic liquid (B) is one or two or more selected from benzyl benzoate, octyl decyl triglyceride, triethyl citrate, dimethyl polysiloxane, cinnamaldehyde, triacetin, piperonyl butoxide, diethyl phthalate, dibutyl phthalate, butyl phthalyl butyl glycolate and ethyl butyrate.

5. A method for producing a particulate formulation in powdery or granular form consisting of the oral pharmaceutical composition according to any one of claims 1 to 3, the method comprising:
preparing a water-soluble polymer aqueous solution by dissolving 100 parts by mass of the water-soluble polymer substance (C) and 1 to 300 parts by mass of the excipient (D) in water and preparing an active ingredient solution by dissolving or dispersing 0.1 to 20 parts by mass of the active ingredient (A) in 1 to 100 parts by mass of the hydrophobic liquid (B);
obtaining a mixed aqueous solution where the hydrophobic liquid (B) comprising the active ingredient (A) is dispersed in said water-soluble polymer aqueous solution by mixing this active ingredient solution into the water-soluble polymer aqueous solution; and
shaping this mixed aqueous solution into particles in powdery or granular form by adding dropwise, spraying or discharging the mixed aqueous solution in a cooling medium having no compatibility with said aqueous solution or in the air followed by drying the particles such that their water content is 1 to 20 mass%.

6. The method for producing a particulate formulation in powdery or granular form according to claim 5, wherein the mixed aqueous solution is made into granules with a particle diameter of 0.5 to 10 mm by adding the mixed aqueous solution dropwise in a cooling medium having no compatibility with said aqueous solution followed by shaping.

7. A method for producing a particulate formulation in powdery or granular form consisting of the oral pharmaceutical composition according to claim 4,
the method comprising:
preparing a water-soluble polymer aqueous solution by dissolving 100 parts by mass of the water-soluble polymer substance (C) and 1 to 300 parts by mass of the excipient (D) in water and preparing an active ingredient solution by dissolving or dispersing 0.00001 parts by mass or more and less than 0.1 parts by mass of the active ingredient (A) in 1 to 100 parts by mass of the hydrophobic liquid (B) ;
obtaining a mixed aqueous solution where the hydrophobic liquid (B) comprising the active ingredient (A) is dispersed in said water-soluble polymer aqueous solution by mixing this active ingredient solution into the water-soluble polymer aqueous solution; and
shaping this mixed aqueous solution into particles in powdery or granular form by adding dropwise, spraying or discharging the mixed aqueous solution in a cooling medium having no compatibility with said aqueous solution or in the air followed by drying the particles such that their water content is 1 to 20 mass%.

8. The method for producing a particulate formulation in powdery or granular form according to claim 7, wherein the mixed aqueous solution is made into granules with a particle diameter of 0.5 to 10 mm by adding the mixed aqueous solution dropwise in a cooling medium having no compatibility with said aqueous solution followed by shaping.

## Patentansprüche

1. Orale pharmazeutische Zusammensetzung, die kein oberflächenaktives Mittel enthält, im Wesentlichen bestehend aus:
0,1 bis 20 Massenteile eines Wirkstoffs (A);
1 bis 100 Massenteile einer hydrophoben Flüssigkeit (B);
100 Massenteile einer wasserlöslichen Polymersubstanz (C); und
1 bis 300 Massenteile eines Hilfsstoffs (D), wobei der Wirkstoff (A) in die hydrophobe Flüssigkeit (B) eingearbeitet ist, und diese hydrophobe Flüssigkeit (B) in einer Zusammensetzung dispergiert ist, die die wasserlösliche Polymersubstanz (C) und den Hilfsstoff (D) umfasst,
wobei der Wirkstoff (A) ein oder zwei oder mehrere in Kombination verwendbare Arzneimittel, die Gegenstand des therapeutischen Drug-Monitoring (TDM) sind, ausgewählt aus Digoxin, Theophyllin, Procainamid, N-Acetylprocainamid, Aprindin, Disopyramid, Lidocain, Pilsicainid, Propafenon, Mexiletin, Flecainid, Chinidin, Cibenzolinsuccinat, Amiodaron, Pirmenol, Bepridil, Phenobarbital, Nitrazepam, Primidon, Diazepam, Phenytoin, Carbamazepin, Zonisamid, Ethosuximid, Acetazolamid, Clobazam, Natriumvalproat, Trimethadion, Clonazepam, Sultiame, Gabapentin, Levetiracetam, Topiramat, Lamotrigin, Gentamicin, Amikacin, Tobramycin, Arbekacin, Vancomycin, Teicoplanin, Voriconazol, Cyclosporin, Tacrolimus, Everolimus, Mycophenolat-Mofetil, Salicylsäure, Methotrexat, Haloperidol, Bromperidol, Lithiumcarbonat und Imatinib, oder ein oder zwei oder mehrere in Kombination verwendbare Krebsmedikamente ist, ausgewählt aus Cyclophosphamid, Ifosfamid, Melphalan, Busulfan, Thiotepa, Nimustin, Ranimustin, Dacarbazin, Procarbazin, Temozolomid, Carmustin, Streptozocin, Bendamustin, Cisplatin, Carboplatin, Oxaliplatin, Nedaplatin, Fluorouracil, Cytarabin, Gemcitabin, Irinotecan, Nogitecan, Doxorubicin, Etoposid, Vinblastin, Vincristin, Vindesin, Vinorelbin, Mitomycin C, Doxorubicin, Epirubicin, Daunorubicin, Bleomycin, Gefitinib, Erlotinib, Afatinib, Dasatinib, Bosutinib, Vandetanib, Sunitinib, Axitinib, Pazopanib, Lenvatinib, Lapatinib, Nintedanib, Nilotinib, Crizotinib, Alectinib, Ruxolitinib, Tofacitinib, Sorafenib, Vemurafenib, Bortezomib, Sirolimus und Temsirolimus, und
wobei die hydrophobe Flüssigkeit (B) eine oder zwei oder mehr ist, ausgewählt aus Benzylbenzoat, Octyldecyltriglycerid, Triethylcitrat, Dimethylpolysiloxan, Zimtaldehyd, Triacetin, Piperonylbutoxid, Diethylphthalat, Dibutylphthalat, Butylphthalylbutylglykolat und Ethylbutyrat.

2. Orale pharmazeutische Zusammensetzung gemäß Anspruch 1, wobei die wasserlösliche Polymersubstanz (C) eine oder zwei oder mehr ist, ausgewählt aus Gelatine, Carrageen, Xanthangummi, Johannisbrotkernmehl und Agar.

3. Orale pharmazeutische Zusammensetzung gemäß einem der Ansprüche 1 bis 2, wobei der Hilfsstoff (D) einer oder zwei oder mehr ist, ausgewählt aus Isomalt, Erythrit, Xylit, Glycerin, Sorbit, Maltit, Mannit, Lactit, reduzierter Palatinose, reduziertem Zuckersirup und pulverisiertem reduziertem Maltosesirup.

4. Orale pharmazeutische Zusammensetzung, die kein oberflächenaktives Mittel enthält, im Wesentlichen bestehend aus:
0,00001 Massenteile oder mehr und weniger als 0,1 Massenteile eines Wirkstoffs (A);
1 bis 100 Massenteile einer hydrophoben Flüssigkeit (B);
100 Massenteilen einer wasserlöslichen Polymersubstanz (C); und
1 bis 300 Massenteile eines Hilfsstoffs (D), wobei der Wirkstoff (A) in die hydrophobe Flüssigkeit (B) eingearbeitet ist und diese hydrophobe Flüssigkeit (B) in einer Zusammensetzung dispergiert ist, die die wasserlösliche Polymersubstanz (C) und den Hilfsstoff (D) umfasst,
wobei der Wirkstoff (A) ein oder zwei oder mehrere in Kombination verwendbare Arzneimittel, die Gegenstand des therapeutischen Drug-Monitoring (TDM) sind, ausgewählt aus Digoxin, Theophyllin, Procainamid, N-Acetylprocainamid, Aprindin, Disopyramid, Lidocain, Pilsicainid, Propafenon, Mexiletin, Flecainid, Chinidin, Cibenzolinsuccinat, Amiodaron, Pirmenol, Bepridil, Phenobarbital, Nitrazepam, Primidon, Diazepam, Phenytoin, Carbamazepin, Zonisamid, Ethosuximid, Acetazolamid, Clobazam, Natriumvalproat, Trimethadion, Clonazepam, Sultiame, Gabapentin, Levetiracetam, Topiramat, Lamotrigin, Gentamicin, Amikacin, Tobramycin, Arbekacin, Vancomycin, Teicoplanin, Voriconazol, Cyclosporin, Tacrolimus, Everolimus, Mycophenolat-Mofetil, Salicylsäure, Methotrexat, Haloperidol, Bromperidol, Lithiumcarbonat und Imatinib, oder ein oder zwei oder mehrere in Kombination verwendbare Krebsmedikamente ist, ausgewählt aus Cyclophosphamid, Ifosfamid, Melphalan, Busulfan, Thiotepa, Nimustin, Ranimustin, Dacarbazin, Procarbazin, Temozolomid, Carmustin, Streptozocin, Bendamustin, Cisplatin, Carboplatin, Oxaliplatin, Nedaplatin, Fluorouracil, Cytarabin, Gemcitabin, Irinotecan, Nogitecan, Doxorubicin, Etoposid, Vinblastin, Vincristin, Vindesin, Vinorelbin, Mitomycin C, Doxorubicin, Epirubicin, Daunorubicin, Bleomycin, Gefitinib, Erlotinib, Afatinib, Dasatinib, Bosutinib, Vandetanib, Sunitinib, Axitinib, Pazopanib, Lenvatinib, Lapatinib, Nintedanib, Nilotinib, Crizotinib, Alectinib, Ruxolitinib, Tofacitinib, Sorafenib, Vemurafenib, Bortezomib, Sirolimus und Temsirolimus, und
wobei die hydrophobe Flüssigkeit (B) eine oder zwei oder mehr ist, ausgewählt aus Benzylbenzoat, Octyldecyltriglycerid, Triethylcitrat, Dimethylpolysiloxan, Zimtaldehyd, Triacetin, Piperonylbutoxid, Diethylphthalat, Dibutylphthalat, Butylphthalylbutylglykolat und Ethylbutyrat.

5. Verfahren zum Herstellen einer partikelförmigen Formulierung in Pulver- oder Granulatform, bestehend aus der oralen pharmazeutischen Zusammensetzung gemäß einem der Ansprüche 1 bis 3, wobei das Verfahren umfasst:
Herstellen einer wässrigen Lösung eines wasserlöslichen Polymers durch Lösen von 100 Massenteile der wasserlöslichen Polymersubstanz (C) und 1 bis 300 Masseteilen des Hilfsstoffs (D) in Wasser und
Herstellen einer Wirkstofflösung durch Lösen oder Dispergieren von 0,1 bis 20 Massenteile des Wirkstoffs (A) in 1 bis 100 Massenteile der hydrophoben Flüssigkeit (B);
Erhalten einer gemischten wässrigen Lösung, in der die hydrophobe Flüssigkeit (B), die den Wirkstoff (A) umfasst, in der wässrigen Lösung des wasserlöslichen Polymers dispergiert ist, durch Mischen dieser Wirkstofflösung in die wässrige Lösung des wasserlöslichen Polymers; und
Formen dieser gemischten wässrigen Lösung in Partikel in Pulver- oder Granulatform durch tropfenweises Zugeben, Versprühen oder Ausstoßen der gemischten wässrigen Lösung in einem Kühlmedium, das mit der wässrigen Lösung nicht kompatibel ist,
oder an der Luft, gefolgt von einem Trocknen der Partikel, so dass ihr Wassergehalt 1 bis 20 Massenprozent beträgt.

6. Verfahren zum Herstellen einer partikelförmigen Formulierung in Pulver- oder Granulatform gemäß Anspruch 5, wobei die gemischte wässrige Lösung durch tropfenweises Zugeben der gemischten wässrigen Lösung in einem Kühlmedium, das mit der wässrigen Lösung nicht kompatibel ist, und anschließendes Formen zu Granulat mit einem Partikeldurchmesser von 0,5 bis 10 mm verarbeitet wird.

7. Verfahren zum Herstellen einer partikelförmigen Formulierung in Pulver- oder Granulatform, bestehend aus der oralen pharmazeutischen Zusammensetzung gemäß Anspruch 4, wobei das Verfahren umfasst:
Herstellen einer wässrigen Lösung eines wasserlöslichen Polymers durch Lösen von 100 Massenteile der wasserlöslichen Polymersubstanz (C) und 1 bis 300 Massenteile des Hilfsstoffs (D) in Wasser und Herstellen einer Wirkstofflösung durch Lösen oder Dispergieren von 0,00001 Massenteile oder mehr und weniger als 0,1 Massenteile des Wirkstoffs (A) in 1 bis 100 Masseteilen der hydrophoben Flüssigkeit (B);
Erhalten einer gemischten wässrigen Lösung, in der die hydrophobe Flüssigkeit (B), die den Wirkstoff (A) umfasst, in der wässrigen Lösung des wasserlöslichen Polymers dispergiert ist, durch Mischen dieser Wirkstofflösung in die wässrige Lösung des wasserlöslichen Polymers; und
Formen dieser gemischten wässrigen Lösung in Partikel in Pulver- oder Granulatform durch tropfenweises Zugeben, Versprühen oder Ausstoßen der gemischten wässrigen Lösung in einem Kühlmedium, das mit der wässrigen Lösung nicht kompatibel ist, oder an der Luft, gefolgt von einem Trocknen der Partikel, so dass ihr Wassergehalt 1 bis 20 Massenprozent beträgt.

8. Verfahren zum Herstellen einer partikelförmigen Formulierung in Pulver- oder Granulatform gemäß Anspruch 7, wobei die gemischte wässrige Lösung durch tropfenweises Zugeben der gemischten wässrigen Lösung in einem Kühlmedium, das mit der wässrigen Lösung nicht kompatibel ist, und anschließendes Formen zu Granulat mit einem Partikeldurchmesser von 0,5 bis 10 mm verarbeitet wird.

## Revendications

1. Composition pharmaceutique orale ne contenant pas de tensioactif constituée essentiellement de :
0,1 à 20 parties en masse d'un ingrédient actif (A) ;
1 à 100 parties en masse d'un liquide hydrophobe (B) ;
100 parties en masse d'une substance polymère soluble dans l'eau (C) ; et
1 à 300 parties en masse d'un excipient (D), dans laquelle l'ingrédient actif (A) est incorporé dans le liquide hydrophobe (B), et ce liquide hydrophobe (B) est dispersé dans une composition comprenant la substance polymère soluble dans l'eau (C) et l'excipient (D),
dans laquelle l'ingrédient actif (A) est un ou deux ou plus utilisables dans une combinaison de médicaments soumis à un suivi thérapeutique des médicaments (TDM) choisis parmi la digoxine, théophylline, procainamide, N-acétylprocaïnamide, aprindine, disopyramide, lidocaïne, pilsicainide, propafénone, mexilétine, flécaïnide, quinidine, succinate de cibenzoline, amiodarone, pirménol, bépridil, phénobarbital, nitrazépam, primidone, diazépam, phénytoïne, carbamazépine, zonisamide, éthosuximide, acétazolamide, clobazam, valproate de sodium, triméthadione, clonazépam, sultiame, gabapentine, lévétiracétam, topiramate, lamotrigine, gentamicine, amikacine, tobramycine, arbékacine, vancomycine, teicoplanine, voriconazole, cyclosporine, tacrolimus, évérolimus, mycophénolate mofétil, acide salicylique, méthotrexate, halopéridol, brompéridol, carbonate de lithium et imatinib, ou un ou deux ou plus utilisables en combinaison d'un médicament anticancéreux choisis parmi le cyclophosphamide, ifosfamide, melphalan, busulfan, thiotépa, nimustine, ranimustine, dacarbazine, procarbazine, témozolomide, carmustine, sreptozocine, bendamustine, cisplatine, carboplatine, oxaliplatine, nédaplatine, fluorouracile, cytarabine, gemcitabine, irinotécan, nogitécan, doxorubicine, étoposide, vinblastine, vincristine, vindesine, vinorelbine, mitomycine C, doxorubicine, épirubicine, daunorubicine, bléomycine, géfitinib, erlotinib, afatinib, dasatinib, bosutinib, vandétanib, sunitinib, axitinib, pazopanib, lenvatinib, lapatinib, nintedanib, nilotinib, crizotinib, alectinib, ruxolitinib, tofacitinib, sorafenib, vémurafénib, bortézomib, sirolimus et temsirolimus, et
dans laquelle le liquide hydrophobe (B) est un ou deux ou plus choisis parmi du benzoate de benzyle, triglycéride d'octyle décyle, citrate de triéthyle, diméthylpolysiloxane, cinnamaldéhyde, triacétine, butoxyde de pipéronyle, phtalate de diéthyle, phtalate de dibutyle, glycolate de butyle phtalyle butyle et butyrate d'éthyle.

2. Composition pharmaceutique orale selon la revendication 1, dans laquelle la substance polymère soluble dans l'eau (C) est un ou deux ou plus choisis parmi de la gélatine, carraghénane, gomme xanthane, gomme de caroube et agar.

3. Composition pharmaceutique orale selon l'une quelconque des revendications 1 à 2, dans laquelle l'excipient (D) est un ou deux ou plus choisis parmi de l'isomalt, érythritol, xylitol, glycérine, sorbitol, maltitol, mannitol, lactitol, palatinose réduit, sirop de sucre réduit et sirop de maltose réduit en poudre.

4. Composition pharmaceutique orale ne contenant pas de tensioactif constituée essentiellement de :
0,00001 partie en masse ou plus et moins de 0,1 partie en masse d'un ingrédient actif (A) ;
1 à 100 parties en masse d'un liquide hydrophobe (B) ;
100 parties en masse d'une substance polymère soluble dans l'eau (C) ; et
1 à 300 parties en masse d'un excipient (D), dans laquelle l'ingrédient actif (A) est incorporé dans le liquide hydrophobe (B), et ce liquide hydrophobe (B) est dispersé dans une composition comprenant la substance polymère soluble dans l'eau (C) et l'excipient (D),
dans laquelle l'ingrédient actif (A) est un ou deux ou plus utilisables dans une combinaison de médicaments soumis à un suivi thérapeutique des médicaments (TDM) choisis parmi la digoxine, théophylline, procaïnamide, N-acétylprocaïnamide, aprindine, disopyramide, lidocaïne, pilsicaïnide, propafénone, mexilétine, flécaïnide, quinidine, succinate de cibenzoline, amiodarone, pirménol, bépridil, phénobarbital, nitrazépam, primidone, diazépam, phénytoïne, carbamazépine, zonisamide, éthosuximide, acétazolamide, clobazam, valproate de sodium, triméthadione, clonazépam, sultiame, gabapentine, lévétiracétam, topiramate, lamotrigine, gentamicine, amikacine, tobramycine, arbékacine, vancomycine, teicoplanine, voriconazole, cyclosporine, tacrolimus, évérolimus, mycophénolate mofétil, acide salicylique, méthotrexate, halopéridol, brompéridol, carbonate de lithium et imatinib ou un ou deux ou plus utilisables en combinaison d'un médicament anticancéreux choisis parmi le cyclophosphamide, ifosfamide, melphalan, busulfan, thiotépa, nimustine, ranimustine, dacarbazine, procarbazine, témozolomide, carmustine, sreptozocine, bendamustine, cisplatine, carboplatine, oxaliplatine, nédaplatine, fluorouracile, cytarabine, gemcitabine, irinotécan, nogitécan, doxorubicine, étoposide, vinblastine, vincristine, vindesine, vinorelbine, mitomycine C, doxorubicine, épirubicine, daunorubicine, bléomycine, géfitinib, erlotinib, afatinib, dasatinib, bosutinib, vandétanib, sunitinib, axitinib, pazopanib, lenvatinib, lapatinib, nintedanib, nilotinib, crizotinib, alectinib, ruxolitinib, tofacitinib, sorafenib, vémurafénib, bortézomib, sirolimus et temsirolimus, et
dans laquelle le liquide hydrophobe (B) est un ou deux ou plus choisis parmi du benzoate de benzyle, triglycéride d'octyle décyle, citrate de triéthyle, diméthylpolysiloxane, cinnamaldéhyde, triacétine, butoxyde de pipéronyle, phtalate de diéthyle, phtalate de dibutyle, glycolate de butyle phtalyle butyle et butyrate d'éthyle.

5. Procédé de production d'une formulation particulaire sous forme poudreuse ou granulaire constituée de la composition pharmaceutique orale selon l'une quelconque des revendications 1 à 3,
le procédé comprenant :
la préparation d'une solution aqueuse de polymère soluble dans l'eau en dissolvant 100 parties en masse de la substance polymère soluble dans l'eau (C) et 1 à 300 parties en masse de l'excipient (D) dans de l'eau et la préparation d'une solution d'ingrédient actif en dissolvant ou en dispersant 0,1 à 20 parties en masse de l'ingrédient actif (A) dans 1 à 100 parties en masse du liquide hydrophobe (B) ;
l'obtention d'une solution aqueuse mixte dans laquelle le liquide hydrophobe (B) comprenant l'ingrédient actif (A) est dispersé dans ladite solution aqueuse de polymère soluble dans l'eau en mélangeant cette solution d'ingrédient actif dans la solution aqueuse de polymère soluble dans l'eau ; et
la mise en forme de cette solution aqueuse mixte en particules sous forme poudreuse ou granulaire par ajout goutte à goutte, pulvérisation ou déversement de la solution aqueuse mixte dans un milieu de refroidissement n'ayant aucune compatibilité avec ladite solution aqueuse ou dans l'air, puis par séchage des particules de telle sorte que leur teneur en eau est de 1 à 20 % en masse.

6. Procédé de production d'une formulation particulaire sous forme poudreuse ou granulaire selon la revendication 5, dans lequel la solution aqueuse mixte est transformée en granulés d'un diamètre de particules de 0,5 à 10 mm par ajout goutte à goutte de la solution aqueuse mixte dans un milieu de refroidissement n'ayant aucune compatibilité avec ladite solution aqueuse suivi d'une mise en forme.

7. Procédé de production d'une formulation particulaire sous forme poudreuse ou granulaire constituée de la composition pharmaceutique orale selon la revendication 4,
le procédé comprenant :
la préparation d'une solution aqueuse de polymère soluble dans l'eau en dissolvant 100 parties en masse de la substance polymère soluble dans l'eau (C) et 1 à 300 parties en masse de l'excipient (D) dans de l'eau et la préparation d'une solution d'ingrédient actif en dissolvant ou en dispersant 0,00001 partie en masse ou plus et moins de 0,1 partie en masse de l'ingrédient actif (A) dans 1 à 100 parties en masse du liquide hydrophobe (B) ;
l'obtention d'une solution aqueuse mixte dans laquelle le liquide hydrophobe (B) comprenant l'ingrédient actif (A) est dispersé dans ladite solution aqueuse de polymère soluble dans l'eau en mélangeant cette solution d'ingrédient actif dans la solution aqueuse de polymère soluble dans l'eau ; et
la mise en forme de cette solution aqueuse mixte en particules sous forme poudreuse ou granulaire par ajout goutte à goutte, pulvérisation ou déversement de la solution aqueuse mixte dans un milieu de refroidissement n'ayant aucune compatibilité avec ladite solution aqueuse ou dans l'air, puis par séchage des particules de telle sorte que leur teneur en eau est de 1 à 20 % en masse.

8. Procédé de production d'une formulation particulaire sous forme poudreuse ou granulaire selon la revendication 7, dans lequel la solution aqueuse mixte est transformée en granulés d'un diamètre de particules de 0,5 à 10 mm par ajout goutte à goutte de la solution aqueuse mixte dans un milieu de refroidissement n'ayant aucune compatibilité avec ladite solution aqueuse suivi d'une mise en forme.
